# EUROPEAN PATENT SPECIFICATION

(11) **EP 1 559 366 B1**
(45) Date of publication and mention of the grant of the patent: **01.10.2014**
(21) Application number: 05001688.0
(22) Date of filing: 27.01.2005
(51) Int. Cl.: A61B 5/0404

(54) **Biological information measurement device**
Vorrichtung zur Messung biologischer Informationen
Dispositif de mesure d'informations biologiques

(30) Priority: 30.01.2004 JP 2004022796
(43) Date of publication of application: 03.08.2005
(73) Proprietor: Omron Healthcare Co., Ltd., Kyoto 617-0002 (JP)
(72) Inventor: Umeda, Masahiro, Muko-shi, Kyoto 617-0002 (JP); Yamamoto, Norihito, Muko-shi, Kyoto 617-0002 (JP); Ishida, Junichi, Muko-shi, Kyoto 617-0002 (JP); Tanabe, Kazuhisa, Muko-shi, Kyoto 617-0002 (JP)
(74) Representative: Kilian Kilian & Partner

(56) References cited:
- WO-A-02/47548
- US-A- 4 742 831
- US-A1- 2003 097 078
- US-B1- 6 363 274
- PATENT ABSTRACTS OF JAPAN vol. 018, no. 553 (C-1263), 21 October 1994 (1994-10-21) & JP 06 197875 A (YOKOGAWA ELECTRIC CORP; others: 02), 19 July 1994 (1994-07-19)

## Description

### BACKGROUND OF THE INVENTION

### Field of the Invention

The present invention relates to a biological information measurement device including an output terminal for establishing connection to an external terminal unit.

### Description of the Background Art

An electrocardiograph and a body fat meter represent examples of widely known biological information measurement devices having a measurement electrode to be brought into contact with a living body and obtaining biological information by processing a biological electric signal detected by the measurement electrode. As to the biological information measurement device, conventionally, connection to an external terminal unit has strongly been demanded.

For example, in the electrocardiograph, demand for analysis and storage of obtained electrocardiographic data, printout of the data on paper medium, and transmission of the electrocardiographic data to a remote location has been very strong, and it has also been demanded to provide an interface in a device main body for the purpose of establishing connection to a personal computer (hereinafter, abbreviated as "PC"), a printer, or communication means. Such demands are great particularly in a Holter electrocardiograph and a portable electrocardiograph that have widely been used in recent days. Specifically, in order to present the electrocardiographic data obtained at home to a specialist, connection to the external terminal unit has strongly been demanded.

In order to realize connection to the external terminal unit in the electrocardiograph, some kind of interface should be provided in the device main body of the electrocardiograph for communicating a signal between the device main body of the electrocardiograph and the external terminal unit. As the interface, a variety of interfaces such as infrared communication, fiber optics communication, magnetic coupling, inductive coupling, and the like may be adopted (see Japanese Patent Laying-Open Nos. 9-224917, 63-206225, and 5-7560, for example). When such an interface is adopted, however, not only a device structure is complicated, but also cost and performance are not satisfactory. Here, as a connection method to realize ensured communication of a signal with low cost, wired connection using a serial bus such as a USB (Universal Serial Bus) and an RS-232C may be employed.

In wired connection using the USB, for example, an output terminal is provided in the device main body of the electrocardiograph and an input terminal is provided in a main body of the external terminal unit. Then, the output terminal and the input terminal are connected to each other via a USB connection cable, so that connection between the device main body of the electrocardiograph and the external terminal unit is established. In this manner, communication of the signal between the device main body of the electrocardiograph and the external terminal unit is reliably realized with low cost.

Meanwhile, when wired connection using a connection cable is adopted, a power supply voltage input to the external terminal unit may be introduced as a surge into a processing circuit provided inside the device main body of the electrocardiograph through the connection cable. If the power supply voltage is introduced while a subject touches the measurement electrode, the subject may receive electric shock through the measurement electrode. Therefore, considering safety of the subject, some measure to prevent electric shock should be provided in order to avoid such an accident of electric shock.

A commonly known measure to prevent electric shock employs a photocoupler (see Japanese Utility Model Laying-Open No. 5-9507, and Japanese Patent Laying-Open Nos. 61-232832 and 63-272324, for example). The photocoupler optically couples two electrically isolated electric circuits with each other, and the photocoupler is normally implemented by a combination of a light-emitting diode and a phototransistor. When the photocoupler is employed, an internal circuit in the external terminal unit can electrically be isolated from the measurement electrode of the electrocardiograph. Therefore, occurrence of an electric shock accident can be prevented.

In order to provide the electric shock prevention measure in the electrocardiograph by using the photocoupler, however, a plurality of photocouplers (two to four) should be mounted on the device main body of the electrocardiograph. The photocoupler is a relatively expensive element, and mounting of a plurality of photocouplers results in increase in manufacturing cost. In addition, larger size of the device is unavoidable.

Meanwhile, the electric shock prevention measure can be provided in the electrocardiograph having the measurement electrode drawn out of the device main body via the connection cable in the following manner. Specifically, such an electrocardiograph may be structured such that simultaneous connection to the device main body of the electrocardiograph, of the connection cable for establishing connection between the device main body of the electrocardiograph and the measurement electrode and the connection cable for establishing connection between the device main body of the electrocardiograph and the external terminal unit is not allowed (see Japanese Utility Model Laying-Open Nos. 5-9508 and 5-9509, and Japanese Patent Laying-Open No. 6-197875, for example). According to such a structure, while the device main body of the electrocardiograph is connected to the external terminal unit, the processing circuit provided inside the device main body of the electrocardiograph is electrically isolated from the measurement electrode in an ensured manner. Therefore, the accident of electric shock as described above can reliably be avoided.

Though such an electric shock prevention measure as above is extremely effective in the electrocardiograph having the measurement electrode drawn out of the device main body through the connection cable, the electrocardiograph having the measurement electrode provided on an outer surface of the device main body (a type mainly found in portable electrocardiographs) cannot adopt such an electric shock prevention measure, and therefore, another measure should be provided.
Document US-B-6 363 274 discloses the most relevant prior art.

A biological information measurement device is known from WO 02/47548 A.

### SUMMARY OF THE INVENTION

An object of the present invention is to provide a biological information measurement device with an electric shock prevention measure that can be adapted without increasing manufacturing cost regardless of its device structure and can reliably prevent occurrence of an accident of electric shock.

The invention is defined in claim 1.

According to such a structure, the switching portion is operated so as to switch electrical connection and disconnection between the measurement electrode and the processing circuit. Accordingly, by electrically disconnecting the measurement electrode from the processing circuit without fail when the external terminal unit is connected to the device main body of the biological information measurement device, an accident of electric shock can reliably be prevented. In addition, as the electric shock prevention measure is implemented by such a simplified structure that the contact point is simply provided between the measurement electrode and the processing circuit, this measure can be adapted to the biological information measurement device having any device structure. Moreover, larger size of the device or increase in manufacturing cost is not caused.

In the biological information measurement device according to the present invention, while a connection terminal for establishing connection to the external terminal unit is connected to the output terminal, the switching portion electrically disconnects the first contact point from the second contact point.

In this manner, while the output terminal provided in the device main body of the biological information measurement device is connected to the connection terminal for establishing connection to the external terminal unit, the switching portion electrically disconnects the first contact point from the second contact point without fail. Thus, further ensured electric shock prevention measure can be provided.

The biological information measurement device according to the present invention further includes a cover provided in the device main body. The cover disallows connection of the connection terminal for establishing connection to the external terminal unit to the output terminal by covering the output terminal when the cover is closed, and allows connection of the connection terminal for establishing connection to the external terminal unit to the output terminal by exposing the output terminal when the cover is opened. The switching portion operates in association with an opening and closing operation of the cover, so as to electrically connect the first contact point to the second contact point when the cover is closed and so as to electrically disconnect the first contact point from the second contact point when the cover is opened.

In this manner, the switching portion is operated in association with the operation of the cover which allows or disallows connection of the connection terminal to the output terminal. Thus, the reliable electric shock prevention measure can be realized based on the operation of the cover by a user.

As described above, according to the present invention, a biological information measurement device with an electric shock prevention measure that can be adapted to the biological information measurement device having any device structure without increasing manufacturing cost and can reliably prevent occurrence of an accident of electric shock can be provided.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic perspective view showing a configuration of a portable electrocardiograph in an embodiment of the present invention.
Fig. 2 is a front view of the portable electrocardiograph in the embodiment of the present invention.
Fig. 3 is a top view of the portable electrocardiograph shown in Fig. 1 when a cover is closed.
Fig. 4 is a bottom view of the portable electrocardiograph in the embodiment of the present invention.
Fig. 5 is a right side view of the portable electrocardiograph in the embodiment of the present invention.
Fig. 6 is a left side view of the portable electrocardiograph in the embodiment of the present invention.
Fig. 7 is a top view of the portable electrocardiograph in the embodiment of the present invention when the cover is opened.
Fig. 8 is a schematic longitudinal cross-sectional view of the portable electrocardiograph in the embodiment of the present invention when the cover is closed.
Fig. 9 is a schematic longitudinal cross-sectional view of the portable electrocardiograph in the embodiment of the present invention when the cover is opened.
Fig. 10 is a perspective view of a measurement posture to be taken by a subject at the time of measuring an electrocardiographic waveform using the portable electrocardiograph in the embodiment of the present invention.
Fig. 11 illustrates a measurement posture to be taken by the subject at the time of measuring an electrocardiographic waveform using the portable electrocardiograph in the embodiment of the present invention, viewed from the above.
Fig. 12 illustrates a state where the portable electrocardiograph in the embodiment of the present invention is held with a right hand.
Fig. 13 is a block diagram showing a state where the portable electrocardiograph in the embodiment of the present invention is connected to a PC.
Fig. 14 is a block diagram illustrating application of the present invention to a Holter electrocardiograph.

### DESCRIPTION OF THE PREFERRED EMBODIMENTS

In the following, one embodiment of the present invention will be described in detail with reference to the drawings. In the embodiment as set forth below, a portable electrocardiograph that can be carried and can easily measure and store an electrocardiographic waveform will be described by way of example of a biological information measurement device.

Initially, an appearance of the portable electrocardiograph in the present embodiment will be described.

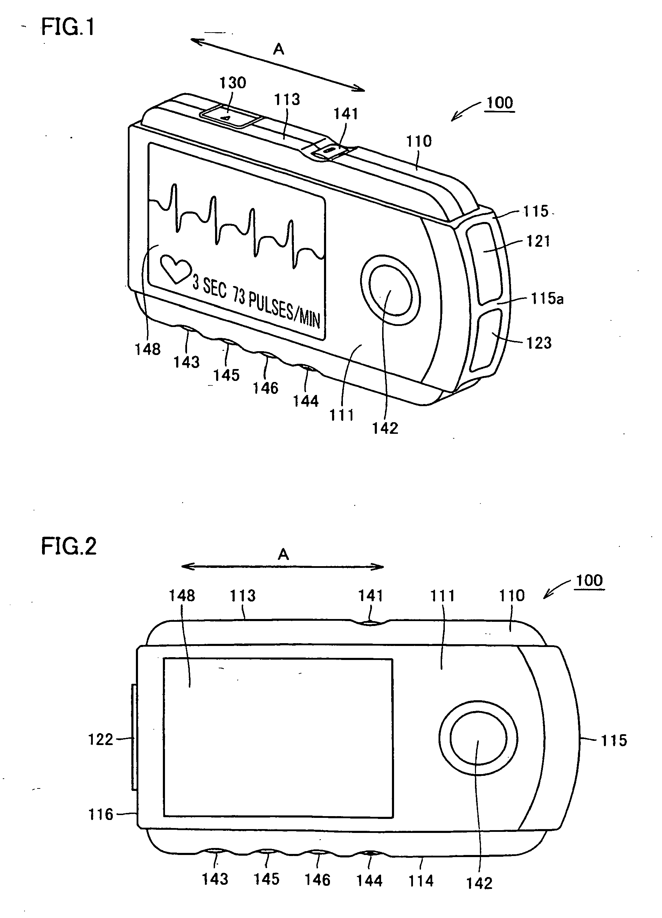

As shown in Figs. 1 to 6, in order to realize excellent usability, a portable electrocardiograph 100 in the present embodiment has such a light weight and a small size that it can be held by one hand. Portable electrocardiograph 100 includes a device main body 110 having a flat and elongated, substantially rectangular parallelepiped shape. On its outer surfaces (a front face 111, a rear face 112, a top face 113, a bottom face 114, a right side face 115, and a left side face 116), a display unit, an operation unit, a measurement electrode, and the like are disposed.

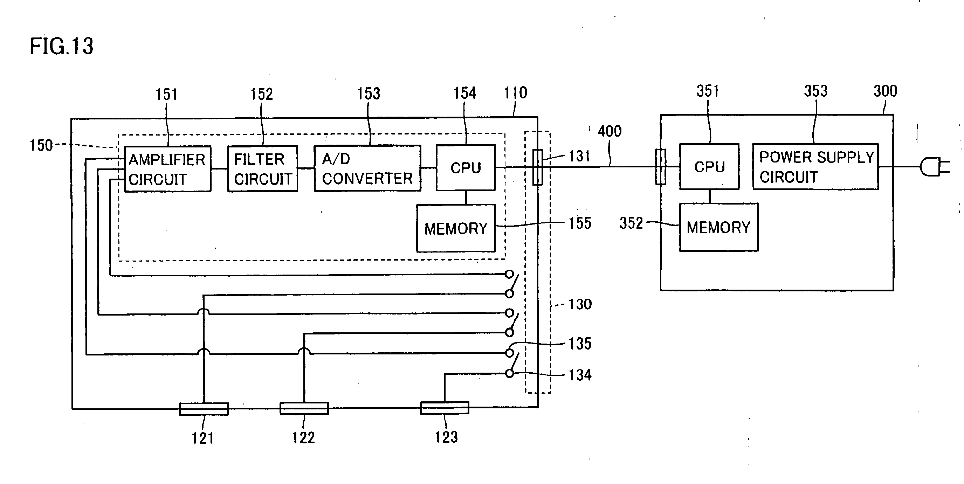

Device main body 110 contains a processing circuit 150 for processing a biological electric signal detected by the measurement electrode (see Fig. 13). Processing circuit 150 includes, for example, an amplifier circuit 151 amplifying the biological electric signal detected by the measurement electrode, a filter circuit 152 removing a noise component from the biological electric signal detected by the measurement electrode, an A/D converter 153 converting an analog signal to a digital signal, a CPU (central processing unit) 154 performing a variety of operations, a memory 155 temporarily storing electrocardiographic information, and the like.

As shown in Figs. 1 and 2, a measurement button 142 serving as an operation button for starting measurement is provided in a portion close to one end in the longitudinal direction (the direction shown with an arrow A in the drawing) of front face 111 of device main body 110. In a portion close to the other end of front face 111 of device main body 110, a display unit 148 is provided. Display unit 148 is implemented, for example, by a liquid crystal display and serves to display a result of measurement or the like. The measurement result is displayed, for example, as electrocardiographic waveforms or numerical data as shown in Fig. 1.

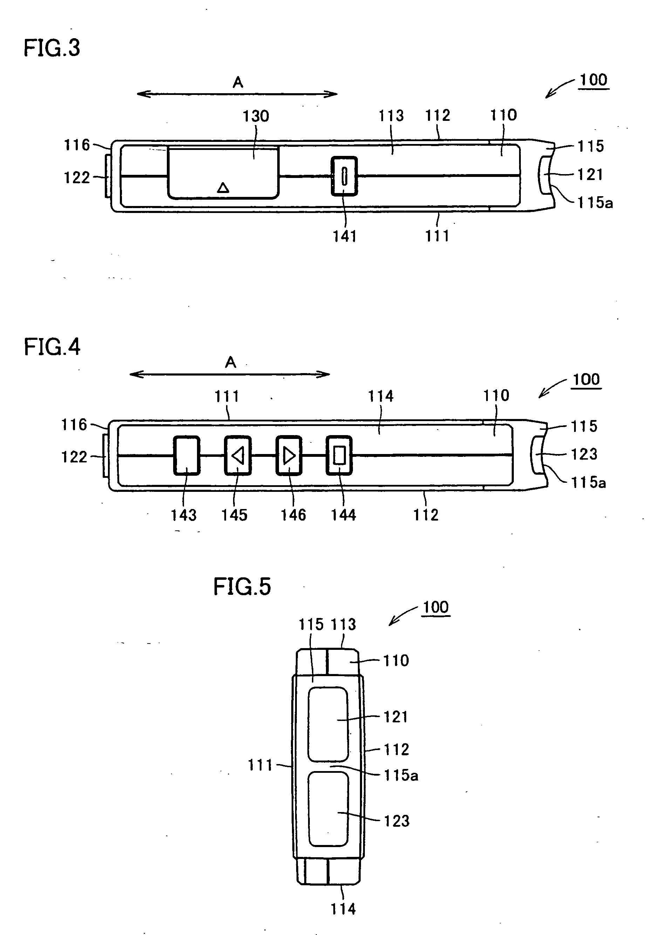

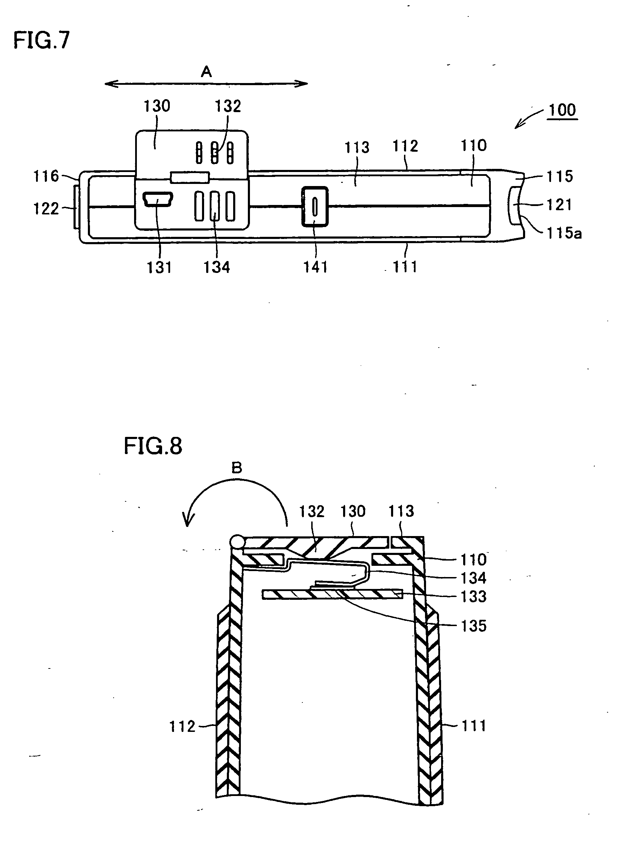

As shown in Figs. 1 and 3, a power button 141 is disposed in a prescribed position in top surface 113 of device main body 110. Power button 141 serves as an operation button for turning ON/OFF portable electrocardiograph 100. A cover 130 is provided in a prescribed position on top face 113 of device main body 110. Cover 130 is provided in order to cover an output terminal 131 (see Figs. 7 to 9) for connection to the external terminal unit which will be described later in a cover-closed state, and cover 130 is attached to device main body 110 such that it is freely opened and closed.

As shown in Figs. 1 and 4, various operation buttons are disposed in prescribed positions on bottom face 114 of device main body 110. In shown portable electrocardiograph 100, a setting button 143, a display button 144, a left scroll button 145, and a right scroll button 146 are disposed. Setting button 143 is used for making a variety of settings for portable electrocardiograph 100, while display button 144 is used for displaying a measurement result on display unit 148. Left scroll button 145 and right scroll button 146 are used for scrolled display of a graph showing a measurement result or guide information displayed on display unit 148.

As shown in Figs. 1 and 5, on right side face 115 located at one end in the longitudinal direction of device main body 110, a negative electrode 121 representing one electrode out of a pair of measurement electrodes as well as an indifferent electrode 123 for deriving a potential serving as a reference in potential variation in the body are disposed. Right side face 115 has a smoothly curved shape, such that a forefinger of the right hand of the subject is fitted thereto when the subject takes a measurement posture which will be described later. In addition, a concave portion 115a extending in an up-down direction is formed in right side face 115. Concave portion 115a is in a shape to receive the forefinger of the right hand of the subject.

Negative electrode 121 and indifferent electrode 123 described above are formed with a conductive member, and electrically connected to first contact points 134 (see Figs. 7 to 9) respectively which will be described later. In addition, negative electrode 121 and indifferent electrode 123 are disposed in concave portion 115a provided in right side face 115 such that their surfaces are exposed on the outer surface of device main body 110. Negative electrode 121 is located closer to top face 113 on right side face 115, while indifferent electrode 123 is located closer to bottom face 114 on right side face 115.

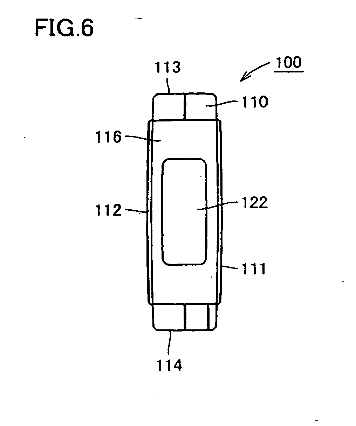

As shown in Fig. 6, on left side face 116 located at the other end in the longitudinal direction of device main body 110, a positive electrode 122 representing the other electrode out of a pair of measurement electrodes is disposed. Positive electrode 122 is formed with a conductive member, and electrically connected to first contact point 134 (see Figs. 7 to 9) which will be described later.

As described above, portable electrocardiograph 100 according to the present embodiment has output terminal 131 for establishing wired connection to the external terminal unit in the prescribed position in top surface 113 of device main body 110. Output terminal 131 is used for supplying the electrocardiographic information temporarily stored in the memory provided inside device main body 110 to the external terminal unit. For the output terminal, a terminal adapted to the USB or RS-232C, for example, is employed.

In the following, output terminal 131 and a structure in its periphery will be described further in detail.

As shown in Figs. 7 to 9, in portable electrocardiograph 100 according to the present embodiment, output terminal 131 for establishing connection to the external terminal unit is provided on the outer surface of device main body 110 in a portion covered with cover 130 described above. In addition, in the vicinity of output terminal 131, first contact points 134 electrically connected to negative electrode 121, positive electrode 122 and indifferent electrode 123 respectively are provided electrically independent of one another. First contact point 134 is formed of a conductive member, and disposed on the outer surface of device main body 110 in a portion covered with cover 130 when the cover is closed, in a manner similar to output terminal 131. As shown in Fig. 7, in an opened state where cover 130 is opened, output terminal 131 and first contact point 134 are exposed on top face 113 of device main body 110.

As described above, cover 130 covers output terminal 131 in a closed state, while it exposes output terminal 131 in the opened state. Therefore, in the closed state, insertion into output terminal 131 of the connection terminal of the connection cable for establishing wired connection to the external terminal unit is not allowed, whereas in the opened state, insertion into output terminal 131 of the connection terminal is allowed.

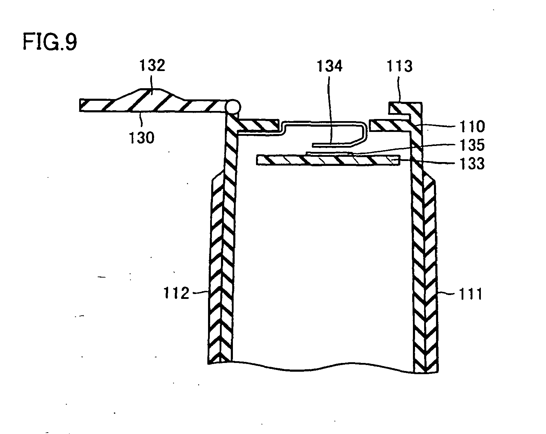

As shown in Fig. 7, a protrusion 132 is provided in a position corresponding to first contact point 134 described above on an inner surface of cover 130. Protrusion 132 is structured such that it abuts on first contact point 134 in the closed state where cover 130 is closed and presses down first contact point 134 by a prescribed amount. As shown in Figs. 8 and 9, a tip end of first contact point 134 is bent like a hook, so that this portion has elasticity.

As shown in Figs. 8 and 9, a second contact point 135 is located below first contact point 134. Second contact points 135 are implemented by conductive patterns formed on a circuit board 133, and provided on circuit board 133 in a manner electrically independent of one another, so as to correspond to respective first contact points 134 provided electrically independent of one another. Second contact points 135 are electrically connected to processing circuit 150 provided inside device main body 110. Here, desirably, a distance between first contact point 134 and second contact point 135 in the opened state is set to a distance sufficient to attain isolation, that is, sufficient to avoid a discharge phenomenon even if a maximum voltage possibly produced between these contact points is applied, considering a possible power supply voltage of the external terminal unit.

As shown in Fig. 8, in the closed state where cover 130 is closed, protrusion 132 provided on the inner surface of cover 130 presses down first contact point 134 toward second contact point 135, whereby first contact point 134 is brought into contact with second contact point 135. In other words, by being pressed by cover 130, first contact point 134 and second contact point 135 are electrically connected to each other.

Meanwhile, as shown in Fig. 9, in order to connect the connection terminal of the connection cable to output terminal 131 provided inside cover 130, cover 130 is pivoted in a direction shown with an arrow B in Fig. 8, so as to attain the opened state where cover 130 is opened. Then, first contact point 134 is no longer pressed by protrusion 132, so that first contact point 134 moves in a direction opposite to second contact point 135 by its elasticity and returns to its original position. In this manner, first contact point 134 does not come in contact with second contact point 135. That is, by avoiding pressing by cover 130, first contact point 134 and second contact point 135 are electrically disconnected from each other.

In this manner, in portable electrocardiograph 100 according to the present embodiment, electrical connection or disconnection between first contact point 134 and second contact point 135 is switched in association with the operation of cover 130. In other words, in portable electrocardiograph 100 according to the present embodiment, cover 130 attains a function as the switching portion. In an electrically connected state, negative electrode 121, positive electrode 122 and indifferent electrode 123 are electrically connected to processing circuit 150 provided inside device main body 110, thereby measurement of an electrocardiographic waveform being allowed. On the other hand, in an electrically disconnected state, negative electrode 121, positive electrode 122 and indifferent electrode 123 are electrically disconnected from processing circuit 150 provided inside device main body 110. Therefore, measurement of an electrocardiographic waveform is not allowed even when negative electrode 121 and positive electrode 122 are pressed against the skin.

Next, a measurement posture to be taken by the subject in measuring an electrocardiographic waveform using portable electrocardiograph 100 having the above-described structure will be described.

During measurement, a subject 200 first closes cover 130 provided in device main body 110. By setting the closed state of cover 130, negative electrode 121, positive electrode 122 and indifferent electrode 123 are electrically connected to processing circuit 150 provided inside device main body 110, and a waiting state in which measurement of the electrocardiographic waveform is allowed is attained.

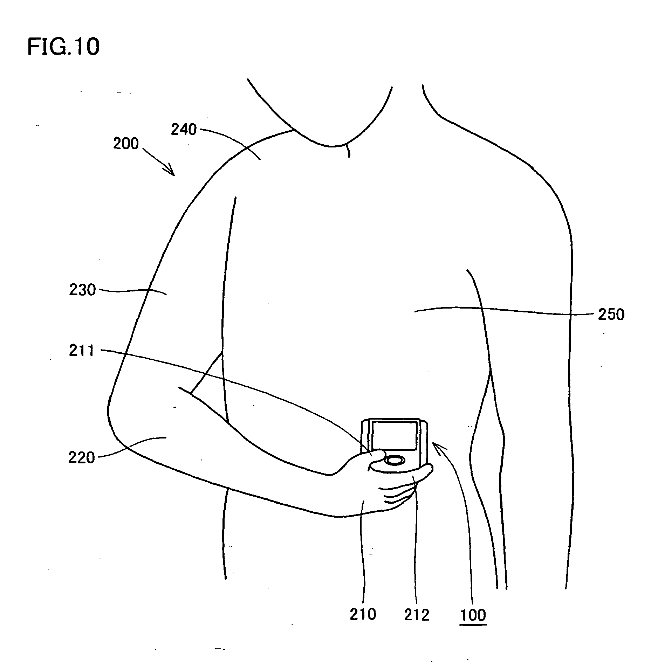

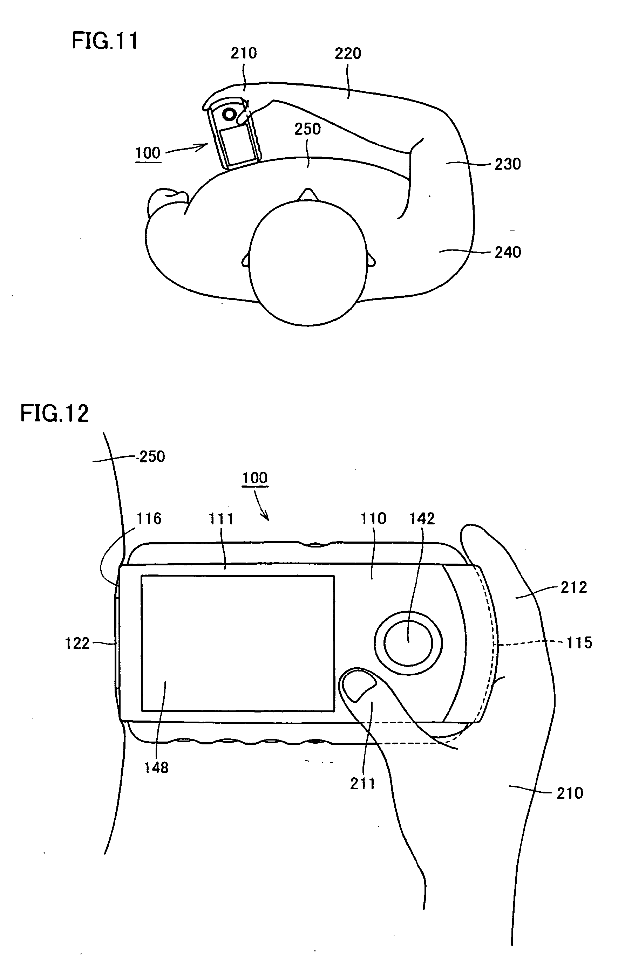

Thereafter, as shown in Figs. 10 and 11, subject 200 holds a portion closer to one end of device main body 110 of portable electrocardiograph 100 with his/her right hand 210, and brings positive electrode 122 provided on left side face 116 located at the other end of device main body 110 into contact with the skin on a fifth intercostal anterior axillary line located in a lower left portion of chest 250. Then, the subject presses measurement button 142 provided on front face 111 of device main body 110 with his/her thumb 211 of right hand 210, and maintains this state for several tens of seconds until measurement of the electrocardiographic waveform is completed.

A state that portable electrocardiograph 100 is held with right hand 210 will now be described.

As shown in Fig. 12, in this measurement posture, subject 200 holds with right hand 210 a portion closer to one end in the longitudinal direction of device main body 110 such that front face 111 of device main body 110 of portable electrocardiograph 100 faces upward. Here, right side face 115 of device main body 110 is covered with forefinger 212 of right hand 210, thumb 211 of right hand 210 is placed on front face 111 of device main body 110, and the middle finger of right hand 210 is placed on the rear face of device main body 110. That is, device main body 110 is held such that it is caught by three fingers. In this state, forefinger 212 of right hand 210 is lightly bent such that the forefinger extends along curved right side face 115 and is inserted in concave portion 115a provided in right side face 115. Forefinger 212 of right hand 210 is thus brought into contact with negative electrode 121 and indifferent electrode 123 provided in concave portion 115a.

When such a measurement posture is taken, negative electrode 121 and indifferent electrode 123 located on right side face 115 of device main body 110 of portable electrocardiograph 100 come in contact with forefinger 212 of right hand 210 of subject 200, and positive electrode 122 located on left side face 116 of device main body 110 comes in contact with chest 250 of subject 200. In this manner, a measurement circuit is implemented in the body of the subject by right hand 210 being in contact with negative electrode 121, forearm 220 without contacting chest 250, a brachium 230 and a right shoulder 240 without contacting chest 250, and chest 250 to which positive electrode 122 is attached, in this order.

As described above, an action potential produced by an activity of cardiac muscle is detected as a potential difference between negative electrode 121 and positive electrode 122 serving as the measurement electrodes, and processed by processing circuit 150 provided inside portable electrocardiograph 100. Electrocardiographic information including the electrocardiographic waveform can thus be obtained. The electrocardiographic information obtained in such a manner is temporarily stored in memory 155 provided inside portable electrocardiograph 100 and output to the external terminal unit when necessary.

A system configuration when the portable electrocardiograph according to the present embodiment is connected to the external terminal unit and the electrocardiographic information stored in the memory is output to the external terminal unit will now be described. It is noted that the system configuration as set forth below employs a PC as the external terminal unit connected to the device main body of the portable electrocardiograph by way of example.

As shown in Fig. 13, when portable electrocardiograph 100 is connected to a PC 300 representing the external terminal unit, cover 130 provided in device main body 110 is opened so as to expose output terminal 131, and the connection terminal located at the other end of a connection cable 400 having one end connected to PC 300 is connected to output terminal 131. Then, device main body 110 of portable electrocardiograph 100 is connected to PC 300 via connection cable 400, and processing circuit 150 provided inside device main body 110 of portable electrocardiograph 100 is electrically connected to an internal circuit of PC 300 (a CPU 351, a memory 352, and the like). In this manner, communication of a signal between processing circuit 150 provided inside device main body 110 of portable electrocardiograph 100 and the internal circuit provided inside PC 300 via connection cable 400 is allowed, so that the electrocardiographic information stored in memory 155 of portable electrocardiograph 100 can be transferred to memory 352 in PC 300.

In portable electrocardiograph 100 according to the present embodiment, by operating cover 130 in order to connect device main body 110 to PC 300, first contact point 134 is electrically disconnected from second contact point 135. Therefore, while device main body 110 is connected to PC 300, negative electrode 121, positive electrode 122 and indifferent electrode 123 are electrically isolated from processing circuit 150 provided inside device main body 110 without fail. Even if a power supply voltage from a power supply circuit 353 in PC 300 should be introduced as surge into processing circuit 150 of portable electrocardiograph 100 through the internal circuit in PC 300 and connection cable 400, the power supply voltage does not reach negative electrode 121, positive electrode 122 and indifferent electrode 123. Therefore, even if the subject touches the measurement electrode when the surge is introduced, there is no possibility of reception of electric shock by the subject.

As described above, in the portable electrocardiograph according to the present embodiment, electrical connection between the measurement electrode and the indifferent electrode and the processing circuit provided inside the device main body is switched between the electrically connected state and the electrically disconnected state, in association with the operation of cover. More specifically, when the cover is opened in order to connect the portable electrocardiograph to the external terminal unit, the first contact point is not in contact with the second contact point. Therefore, while the device main body of the portable electrocardiograph is connected to the external terminal unit, the measurement electrode and the indifferent electrode are electrically isolated from the processing circuit provided inside the device main body without fail. Therefore, an electric shock accident that may take place when the portable electrocardiograph is connected to the external terminal unit can reliably be prevented.

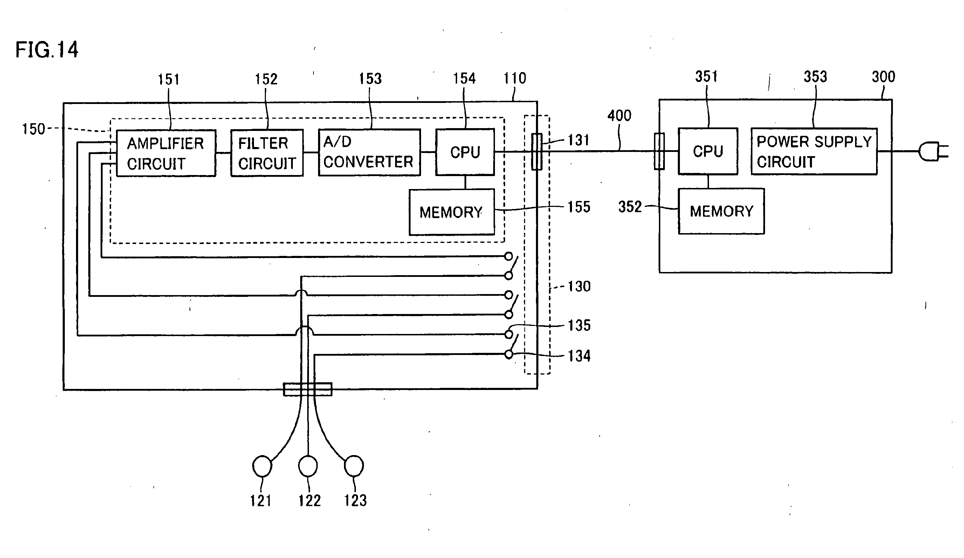

In addition, a structure to prevent electric shock as above can be implemented by such a simplified structure that the contact point is simply provided between the measurement electrode and the processing circuit. Therefore, this structure can be adapted to the biological information measurement device having any device structure. For example, as shown in Fig. 14, the present invention is applicable to a Holter electrocardiograph. Since the electric shock prevention measure is realized with the simplified structure that the contact point is simply provided between the measurement electrode and the processing circuit, larger size of the device or increase in manufacturing cost is not caused.

The cover is attached to the device main body such that the cover is freely opened and closed. The cover is attached to the device main body in a manner slidable with respect to the device main body.

In addition, in the present embodiment described above, an example in which electrical connection or disconnection between the first contact point and the second contact point is switched in association with the opening and closing operation of the cover has been described. The present invention, however, is not limited to such an example. For example, electrical connection or disconnection between the first contact point and the second contact point may be switched in association with an operation to connect the connection terminal to the output terminal. Alternatively, a switch simply switching electrical connection and disconnection between the first contact point and the second contact point may separately be provided in the device main body, without being associated with a connection operation by the user in particular.

Moreover, in the present embodiment described above, an example in which the signal is output solely from the portable electrocardiograph to the external terminal unit has been described. It is naturally possible, however, to attain a structure allowing input and output of the signal in a bidirectional manner. In addition, the external terminal unit to be connected is not limited to the PC, and a variety of terminal units such as a printer and a communication device are applicable.

Furthermore, in the present embodiment described above, the present invention has been applied to the portable electrocardiograph having the measurement electrode and the indifferent electrode provided on the outer surface of the device main body and the Holter electrocardiograph having the measurement electrode and the indifferent electrode drawn out of the device main body by way of example. The present invention, however, may naturally be applied to a stationary electrocardiograph installed in a hospital, for example. In addition, an application of the present invention is not limited to the electrocardiograph. The present invention is applicable to any biological information measurement device having a measurement electrode to be brought into contact with a living body and obtaining biological information by processing a biological electric signal detected by the measurement electrode. Examples of the biological information measurement device include a measurement device to measure a myoelectric potential, brain wave, living body impedance (impedance of internal tissue or skin), or the like.

Although the present invention has been described and illustrated in detail, it is clearly understood that the same is by way of illustration and example only and is not to be taken by way of limitation, the scope of the present invention being limited only by the terms of the appended claims.

## Claims

1. A biological information measurement device, comprising:
a measurement electrode (122) adapted to be brought into contact with a living body;
a processing circuit (150) provided inside a device main body (110) and adapted to process a biological electric signal detected by said measurement electrode (122);
an output terminal (131) provided in said device main body (110) and adapted to supply biological Information obtained by processing in said processing circuit (150) to an external terminal unit;
a first contact point (134) provided in said device main body (110) and electrically connected to said measurement electrode (122);
a second contact point (135) provided in said device main body (110) and electrically connected to said processing circuit (150); and
switching means provided in said device main body (110) for switching electrical connection and disconnection between said first contact point (134) and said second contact point (135), wherein said switching means is adapted to electrically disconnect said first contact point (134) from said second contact point (135) while a connection terminal for establishing connection to the external terminal unit is connected to said output terminal (131),
a cover (130) which is provided in and is slidably attached to said device main body (110), wherein
said cover (130) is adapted to disallow connection of the connection terminal for establishing connection to the external terminal unit to said output terminal (131) by covering said output terminal (131) when said cover (130) is closed, and to allow connection of the connection terminal for establishing connection to the external terminal unit to said output terminal (131) by exposing said output terminal (131) when said cover is opened, and
said switching means is adapted to operate in association with an opening and closing operation of said cover (130), so as to electrically connect said first contact point (134) to said second contact point (135) when the cover is closed and so as to electrically disconnect said first contact point (134) from said second contact point (135) when the cover is opened.

2. The biological information measurement device according to claim 1, wherein
said measurement electrode (122) is provided on an outer surface (116) of said device main body (110).

## Patentansprüche

1. Vorrichtung zum Messen biologischer Informationen, aufweisend:
eine Messelektrode (122), welche eingerichtet ist, mit einem lebenden Körper in Berührung gebracht zu werden;
eine Verarbeitungsschaltung (150), welche innerhalb eines Vorrichtungshauptkörpers (110) vorgesehen und eingerichtet ist, ein biologisches elektrisches Signal, welches von der Messelektrode (122) erfasst wird, zu verarbeiten;
einen Ausgangsanschluss (131), welcher auf dem Vorrichtungshauptkörper (110) vorgesehen und eingerichtet ist, biologische Informationen, welche durch die Verarbeitung in der Verarbeitungsschaltung (150) erhalten werden, einer externen Anschlusseinheit zuzuführen;
einen ersten Kontaktpunkt (134), welcher in dem Vorrichtungshauptkörper (110) vorgesehen und elektrisch mit der Messelektrode (122) verbunden ist;
einen zweiten Kontaktpunkt (135), welcher in dem Vorrichtungshauptkörper (110) vorgesehen und elektrisch mit der Verarbeitungsschaltung (150) verbunden ist; und
eine in dem Vorrichtungshauptkörper (110) vorgesehene Schalteinrichtung zum Umschalten zwischen einer elektrischen Verbindung und Trennung zwischen dem ersten Kontaktpunkt (134) und dem zweiten Kontaktpunkt (135), wobei die Schalteinrichtung eingerichtet ist, den ersten Kontaktpunkt (134) von dem zweiten Kontaktpunkt (135) elektrisch zu trennen, während ein Verbindungsanschluss zum Herstellen einer Verbindung mit der externen Anschlusseinheit mit dem Ausgangsanschluss (131) verbunden ist,
eine Abdeckung (130), welche in dem Vorrichtungshauptkörper (110) vorgesehen und mit diesem verschiebbar verbunden ist, wobei
die Abdeckung (130) eingerichtet ist, eine Verbindung des Verbindungsanschlusses zum Herstellen einer Verbindung mit der externen Anschlusseinheit mit dem Ausgangsanschluss (131) durch Abdecken des Ausgangsanschlusses (131) nicht zuzulassen, wenn die Abdeckung (130) geschlossen ist, und eine Verbindung des Verbindungsanschlusses zum Herstellen einer Verbindung mit der externen Anschlusseinheit mit dem Ausgangsanschluss (131) durch Freigeben des Ausgangsanschlusses (131) zuzulassen, wenn die Abdeckung geöffnet ist, und wobei
die Schalteinrichtung eingerichtet ist, in Verbindung mit einem Öffnungs- und Schließvorgang der Abdeckung (130) betrieben zu werden, um den ersten Kontaktpunkt (134) mit dem zweiten Kontaktpunkt (135) elektrisch zu verbinden, wenn die Abdeckung geschlossen ist, und um den ersten Kontaktpunkt (134) von dem zweiten Kontaktpunkt (135) elektrisch zu trennen, wenn die Abdeckung geöffnet ist.

2. Vorrichtung zum Messen biologischer Informationen gemäß Anspruch 1, wobei
die Messelektrode (122) auf einer Außenfläche (116) des Vorrichtungshauptkörpers (110) vorgesehen ist.

## Revendications

1. Dispositif de mesure d'informations biologiques, comprenant :
une électrode de mesure (122) adaptée pour être amenée en contact avec un corps vivant ;
un circuit de traitement (150) prévu à l'intérieur d'un corps principal (110) de dispositif et adapté pour traiter un signal électrique biologique détecté par ladite électrode de mesure (122) ;
un terminal de sortie (131) prévu dans ledit corps principal (110) de dispositif et adapté pour fournir les informations biologiques obtenues par traitement dans ledit circuit de traitement (150) à une unité de terminal externe ;
un premier point de contact (134) prévu dans ledit corps principal (110) de dispositif et électriquement raccordé à ladite électrode de mesure (122) ;
un second point de contact (135) prévu dans ledit corps principal (110) de dispositif et électriquement raccordé audit circuit de traitement (150) ; et
des moyens de commutation prévus dans ledit corps principal (110) de dispositif pour commuter la connexion électrique et la déconnexion entre ledit premier point de contact (134) et ledit second point de contact (135), dans lequel lesdits moyens de commutation sont adaptés pour déconnecter électriquement ledit premier point de contact (134) dudit second point de contact (135) alors qu'un terminal de connexion pour établir la connexion avec l'unité de terminal externe est raccordé audit terminal de sortie (131),
un couvercle (130) qui est prévu dans et est fixé de manière coulissante audit corps principal (110) de dispositif, dans lequel :
ledit couvercle (130) est adapté pour interdire la connexion du terminal de connexion prévu pour établir la connexion avec l'unité de terminal externe, avec ledit terminal de sortie (131) en recouvrant ledit terminal de sortie (131) lorsque ledit couvercle (130) est fermé, et pour permettre la connexion du terminal de connexion prévu pour établir la connexion avec l'unité de terminal externe, avec ledit terminal de sortie (131) en exposant ledit terminal de sortie (131) lorsque ledit couvercle est ouvert, et
lesdits moyens de commutation sont adaptés pour fonctionner en association avec une opération d'ouverture et de fermeture dudit couvercle (130), afin de connecter électriquement ledit premier point de contact (134) audit second point de contact (135) lorsque le couvercle est fermé et afin de déconnecter électriquement ledit premier point de contact (134) dudit second point de contact (135) lorsque le couvercle est ouvert.

2. Dispositif de mesure d'informations biologiques selon la revendication 1, dans lequel :
ladite électrode de mesure (122) est prévue sur une surface externe (116) dudit corps principal (110) de dispositif.
